# EUROPÄISCHE PATENTSCHRIFT

(11) **EP 1 850 801 B1**
(45) Veröffentlichungstag und Bekanntmachung des Hinweises auf die Patenterteilung: **22.02.2012**
(21) Anmeldenummer: 06707302.3
(22) Anmeldetag: 27.02.2006
(51) Int. Cl.: A61F 2/36, C22C 14/00, C22F 1/18

(54) **GELENKPROTHESE AUS EINER TITAN-MOLYBDÄN-LEGIERUNG**
JOINT PROSTHESIS MADE OF A TITANIUM-MOLYBDENUM-ALLOY
PROTHÈSE ARTICULAIRE D'UN ALLIAGE DE TITANE ET MOLYBDÈNE

(30) Priorität: 25.02.2005 EP 05004178
(43) Veröffentlichungstag der Anmeldung: 07.11.2007
(73) Patentinhaber: WALDEMAR LINK GmbH & Co. KG, 22339 Hamburg (DE)
(72) Erfinder: BALIKTAY, Sevki, 14050 Berlin (DE); KELLER, Arnold, 23863 Kayhude (DE)
(74) Vertreter: Glawe, Delfs, Moll
(86) Internationale Anmeldenummer: PCT/EP2006/001791
(87) Internationale Veröffentlichungsnummer: WO 2006/089791

(56) Entgegenhaltungen:
- US-A1- 2004 136 859
- US-B1- 6 238 491
- DOANCHIE ET AL: "Titanium, a technical guide" Dezember 2000 (2000-12), ASM INTERNATIONAL , USA , XP002336954 ISBN: 0-87170-686-5 Seite 39 - Seite 42

## Beschreibung

Die Erfindung betrifft eine Gelenkprothese mit einem Schaft aus einer Titanlegierung.

Die großen Gelenke des menschlichen Körpers sind mechanisch hoch belastet. So müssen die Gelenke des Bewegungsapparats einen Großteil des Körpergewichts tragen, zudem werden sie bei jedem Schritt bewegt. Daher weisen die die Gelenke tragenden Knochen eine kräftige Kortikalisstruktur auf. Ihre Integrität ist wichtig für eine ausreichende Funktion des Gelenks. Entsprechendes gilt für die Gelenke der Arme. Bei ihnen ist zwar die Gewichtsbelastung niedriger, jedoch werden sie häufiger bewegt und daher einem beträchtlichen Verschleiß ausgesetzt. Zudem sind sie schwächer dimensioniert und anfällig gegenüber Verletzungen.

Zur dauerhaften Implantation vorgesehene Prothesen (Endoprothesen) müssen nicht nur ausreichende mechanische Eigenschaften zur Sicherstellung der gewünschten Funktionalität haben, sondern sie müssen auch eine möglichst hohe Biokompatibilität aufweisen, damit sie über einen langen Zeitraum hinweg vom Patienten toleriert werden. Gerade der letztere Aspekt ist von ausgesprochen hoher Wichtigkeit, da eventuell auftretende Unverträglichkeiten meist eine Explantation der Prothese erforderlich machen. Das kommt einem Ausfall der Prothese gleich.

Es ist bekannt, dass eine unzureichende Lastüberleitung von der Prothese zum umgebenden Knochen zu einer Degeneration des Knochengewebes führen kann. Das hat nicht selten eine Lockerung der Prothese zur Folge. Zur vermeidung dieser Degeneration ist es daher wichtig, für eine möglichst physiologische Belastung durch die Prothese zu sorgen. Untersuchungen haben gezeigt, dass Hüftprothesen mit niedrigerem Elastizitätsmodul eine Belastungssituation erzeugen, die physiologischer ist als bei steifen Prothesen. So wurde beispielsweise bei Femurprothesen von Kobaltchrom-Legierungen, die durchweg einen sehr hohen Elastizitätsmodul im Bereich von ca. 200.000 N/mm² aufweisen, auf Titanlegierungen mit einem niedrigeren Elastizitätsmodul übergegangen, wie zum Beispiel TiA16V4 mit ca. 100.000 N/mm². Diese Werte liegen aber immer noch beträchtlich über dem Elastizitätsmodul des kortikalen Knochens von etwa 25.000 N/mm².

Aus US 2004/0136859 A1 ist es bekannt, dass medizinische Implantate aus β-Titanlegierungen gegossen werden können.

Der Erfindung liegt die Aufgabe zugrunde, eine Gelenkprothese der eingangs genannten Art dahingehend zu verbessern, dass eine physiologischere Lastübertragung erreicht wird.

Die erfindungsgemäße Lösung liegt in einer Gelenkprothese mit den Merkmalen der unabhängigen Ansprüche. Vorteilhafte Weiterbildungen sind Gegenstand der Unteransprüche.

Erfindungsgemäß ist bei einer Gelenkprothese mit einem Schaft aus einer Titanlegierung vorgesehen, dass zumindest der Schaft feingegossen ist und eine mittenzentrierte kubische Kristallstruktur (sog. β-Titanlegierung) aufweist. Die Titanlegierung ist eine Titan-Molybdän-Legierung mit einem Molybdängehalt von 15%, Rest Titan. Der Elastizitätsmodul des Schafts beträgt 59,4 kN/mm² bis 68 kN/mm².

Es hat sich gezeigt, dass mit der erfindungsgemäßen Gelenkprothese ein deutlich geringerer Elastizitätsmodul erreicht werden kann. Es können, abhängig von der vorgenommenen Wärmebehandlung, Werte von ca. 60.000 N/mm² erreicht werden. Das entspricht nahezu einer Halbierung des bisher mit Titanlegierungen erreichten Elastizitätsmoduls. Weiter sieht die Erfindung vor, dass zumindest der Schaft feingegossen ist. Das ermöglicht eine komplexere Formgebung der Prothese. Die bisher bei Prothesen aus Titan hauptsächlich zur Anwendung kommenden Schmiedeverfahren erlauben nur die Herstellung vergleichsweise einfacher Strukturen. Diese Beschränkung wird dank der Erfindung überwunden. Die Prothesen gemäß der Erfindung können damit besser an die aufzunehmenden Lasten angepasst sein. So kann die Formgebung der Prothese feiner je nach den jeweiligen lokalen Belastungen variieren. Die Prothese braucht nur in genau den Bereichen stärker und damit steifer dimensioniert zu sein, in denen hohe Belastungen auftreten; in den übrigen kann sie schwächer und damit elastischer ausgebildet sein. Dies ermöglicht eine weitere Verbesserung der Anpassung der Prothese an die anatomischen Gegebenheiten. Außerdem können Befestigungselemente wie Vorsprünge auf einfache Weise integral mit der Prothese ausgeführt sein. Es können mehr und komplexere Befestigungselemente vorgesehen sein. Die Prothese eignet sich damit besser für eine zementlose Implantation. Es ist das Verdienst der Erfindung, dass solch komplexe Formen, die durch Schmiedeverfahren praktisch nicht zu realisieren sind, auch bei Prothesen aus β-Titanlegierungen erreicht werden können. Im Regelfall wird es so sein, dass die Prothese mitsamt dem Schaft in einem Stück feingegossen und wärmebehandelt wird, jedoch soll es nicht grundsätzlich ausgeschlossen sein, die Prothese aus mehreren Teilen einschließlich den Schaft zu fügen.

Mit Vorteil kann die Erfindung für künstliche Hüftgelenke verwendet werden, und zwar insbesondere bei Femurprothesen. Diese zählen zu den am höchsten belasteten Prothesen, und sie weisen einen kompliziert geformten Schaft zur Implantation im Femur auf. Es hat sich gezeigt, dass gerade im oberen Bereich des Femur es leicht zu Degenerationserscheinungen kommt, wenn eine zu steife Prothese implantiert ist. Ein Ausfall der Prothese ist die häufige Folge. Bei einer Femurprothese gemäß der Erfindung ist der Elastizitätsmodul deutlich geringer und damit viel näher an einem physiologischen Wert des Knochenmaterials im oberen Bereich des Femur. Der Gefahr einer Degeneration wird mit der erfindungsgemäßen Femurprothese erfolgreich entgegengewirkt. Entsprechendes gilt für eine Ausführung als Knieprothese, die in der Regel recht lange Schäfte aufweisen.

Die Titanlegierung ist eine Titan-Molybdänlegierung. Durch den Zusatz von Molybdän wird eine Stabilisierung der sog. β-Phase der Titanlegierung erreicht. Sie ermöglicht die Ausbildung der gewünschten mittenzentrierten kubischen Kristallstruktur. Molybdän als Legierungselement weist gegenüber anderen Legierungselementen, die ebenfalls eine Stabilisierung der β-Phase bewirken, insbesondere Niob oder Vanadium, eine geringere Toxizität auf. Die Verringerung der Toxizität ist für eine zur Langzeitimplantation vorgesehene Prothese ein gewichtiger Vorteil.

Der Anteil des Molybdäns in der Legierung liegt bei 15%. Damit ergibt sich eine ausreichende Stabilisierung der β-Phase bis in den Bereich der Raumtemperatur. Damit kann durch schnelles Abkühlen nach dem Gießen eine metastabile β-Phase erreicht werden. Die mittlere Korngröße der Kristallstruktur beträgt dabei mindestens 0,3 mm, vorzugsweise 0,5 mm. Die Zugabe weiterer Legierungsbildner ist entbehrlich. Insbesondere ist es nicht erforderlich, dass Vanadium oder Aluminium hinzugefügt ist. Der Verzicht darauf hat den bereits angesprochenen Vorteil, dass die von diesen Legierungsbildnern ausgehende Toxizität vermieden werden kann. Entsprechendes gilt für Bismut, das in seiner Biokompatibilität ebenfalls nicht an Titan heranreicht. Ferner hat die Titan-Molybdänlegierung den Vorteil, gegenüber bekannten Legierungen wie TiA16V4 ein verbessertes Formfüllungsvermögen aufzuweisen. Es ist damit ermöglicht, scharfkantigere Strukturen im Feingießverfahren zu bilden.

Besonders bewährt ist es, wenn zumindest der Schaft der erfindungsgemäßen Prothese heißisostatisch gepresst und lösungsgeglüht ist. Es hat sich gezeigt, dass bei einem in dieser Weise wärmebehandelten Material erhebliche Verbesserungen in Bezug auf Sprödigkeit erreicht werden. Durch das heißisostatisch Pressen wird ungünstigen Effekten einer Anreicherung des Molybdäns in Dendriten unter Verarmung der Restschmelze entgegengewirkt, indem interdendritische Ausscheidungen in Lösung gebracht werden. Günstig ist eine Temperatur unterhalb der β-Transustemperatur, und zwar bis zu 100 °C darunter. Für eine Titanmolybdänlegierung mit 15 % Molybdänanteil haben sich Temperaturen im Bereich von 710 °C bis 760 °C, vorzugsweise von etwa 740 °C. bewährt. Mit dem Lösungsglühen wird eine Verbesserung der Duktilität der Legierung erreicht. Bewährt haben sich hierfür Temperaturen von mindestens 700 °C bis zu 880 °C, vorzugsweise im Bereich von 800 °C bis 860 °C. Eine vorauslagerung vor oder nach dem heißisostatischen Pressen ist dabei nicht erforderlich. Zur Abkühlung nach dem Lösungsglühen ist der Schaft zweckmäßigerweise mit Wasser abgeschreckt.

Die Erfindung wird nachfolgend unter Bezugnahme auf die Zeichnung erläutert, in der ein vorteilhaftes Ausführungsbeispiel dargestellt ist. Es zeigen:
- Fi. 1: eine schematische Ansicht eines ersten Ausführungsbeispiels einer erfindungsgemäßen Gelenkprothese;
- Fi. 2: eine schematische Ansicht eines weiteren Ausführungsbeispiels einer erfindungsgemäßen Gelenkprothese;
- Fi. 3: eine Abbildung der Kristallstruktur unmittelbar nach dem Feingießen (1000fach vergrößert);
- Fi. 4: eine Abbildung der Kristallstruktur nach heißisostatischen Pressen und Lösungsglühen; und
- Fi. 5: eine Tabelle mit mechanischen Eigenschaften der erfindungsgemäßen Prothese.

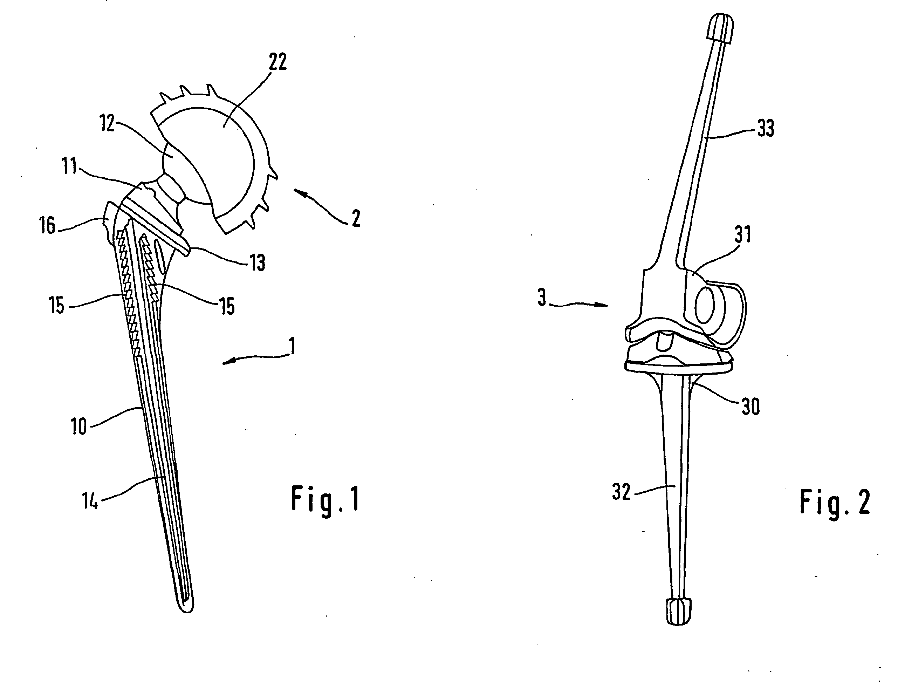

Das in Fig. 1 dargestellte Ausführungsbeispiel zeigt eine Femurprothese für ein künstliches Hüftgelenk. Die Femurprothese 1 besteht aus einer β-Titanlegierung, nämlich TiMo15. Diese Legierung weist bei Raumtemperatur eine mittenzentrierte kubische Kristallstruktur auf.

Die Femurprothese 1 ist zur Implantation am oberen Ende des Femur vorgesehen. Sie kann mit einer im Beckenknochen implantierten Acetabulumkomponente 2 zusammenwirken. Die Femurprothese 1 weist einen langgestreckten Schaft 10 als Knochenverankerungselement und einen sich stumpfwinklig anschließenden Hals 11 auf. An seinem schaftfernen Ende ist ein Gelenkkopf 12 angeordnet, der zusammen mit einem Lagereinsatz 22 der Acetabulumkomponente 2 ein Kugelgelenk bildet. Zur Implantation erfolgt eine vollständige oder teilweise Resektion des Oberschenkelhalses mit seinem Kopf, und ein Zugang zur Markhöhle des Femur wird eröffnet. Über diesen Zugang wird der Schaft 10 der Femurprothese 1 in die Markhöhle eingeführt und dort verankert. Je nach Ausführung ist Zement als Verankerungsmittel vorgesehen, oder die Fixierung erfolgt zementlos.

Die Femurprothese 1 leitet auf das Hüftgelenk einwirkende mechanische Belastungen, seien es statische beim Stehen oder dynamische beim Laufen, in den Femur ein. Für eine dauerhafte sichere Verankerung der Femurprothese 1 im Knochenmaterial des Femur ist eine physiologisch günstige Belastungsübertragung wichtig. Ist die Femurprothese 1 sehr steif ausgeführt, übernimmt sie einen Großteil der Belastung und entlastet somit das Knochenmaterial insbesondere im oberen Bereich des Femur. Das führt auf längere Sicht zu einer Degeneration des Femur in diesem Bereich. Als Folge droht eine Lockerung der Femurprothese 1, schließlich ein Ausfall der Prothese. Um diesen Ausfallmodus zu verhindern, ist es an sich bekannt, die Femurprothese 1 weniger steif, also elastischer mit einem physiologisch günstigen niedrigen Elastizitätsmodul auszuführen. Insbesondere der Schaft 10 der Femurprothese 1 ist insoweit kritisch. Das Knochenmaterial des Femur weist in dem kortikalen Bereich einen Elastizitätsmodul von ca. 20.000 bis 25.000 N/mm² auf. Die Femurprothese-1 weist erfindungsgemäß einen Elastizitätsmodul von ca. 60.000 N/mm² auf. Das ist ein günstiger Wert, er liegt deutlich niedriger als derjenige von herkömmlicherweise verwendeten Materialien wie TiA16V4. Diese weisen einen Elastizitätsmodul von ca. 100.000 N/mm² oder gar 200.000 N/mm² bei Kobalt-Chromlegierungen auf.

Dank der Erfindung können im Feinguss auch komplexe Formen leicht hergestellt werden. So weist die Femurprothese 1 an ihrem Schaft 10 eine Vielzahl von Ausnehmungen und sägezahnartigen Vorsprüngen auf. Sie dienen einer verbesserten Verankerung der Femurprothese 1 in dem Femur, und ermöglichen eine zementlose Implantation. Vorgesehen sind mehrere in Längsrichtung des Schafts 10 verlaufende Rillen 14. Sie sind sowohl auf der anterioren und posterioren Seite des Schafts 10 angeordnet, können aber auch an den Lateralseiten vorgesehen sein. Im oberen Bereich des Schafts 10 sind mehrere Reihen mit Sägezahnvorsprüngen 15 vorgesehen. Weiter ist am Übergang zum Hals 11 ein umlaufender Ring 13 vorgesehen. Er kann als gesondertes Element ausgeführt sein, dank der Erfindung kann er aber auch einstückig mit dem Schaft 10 und Hals 11 sein. Generell ist eine einstückige Ausführung der Prothese vorzuziehen, mit Ausnahme von auswechselbaren oder optionalen Anbau- oder Verschleißteilen. Ferner ist am Schaft 10 ein an den Ring 13 angrenzender Fixiervorsprung 16 als Drehsicherung vorgesehen. Derart komplexe Formen aufweisende Gelenkprothesen können herkömmlicherweise nur aus TiA16V4 hergestellt werden. Dieses weist aber eine andere, ungünstigere Kristallstruktur und damit einen unerwünscht hohen Elastizitätsmodul auf.

Die Erfindung kann mit Vorteil auch bei anderen Arten von Gelenkprothesen Verwendung finden. In Fig. 2 ist als ein weiteres Ausführungsbeispiel eine Knieprothese 3 dargestellt. Sie umfasst eine Femurkomponente 31 und eine Tibiakomponente 30. Die Femurkomponente 31 weist einen langen Schaft 33 als Knochenverankerungselement auf. Er ist zur Implantation in den durch Sektion des natürlichen Kniegelenks geöffneten Markkanal des Femur ausgebildet. Wie auch im Fall der Femurprothese, so stellt sich auch hier das Problem einer Degeneration der umgebenden Kortikalisstruktur bei einer zu steifen Ausführung der Knieprothese 3, insbesondere ihres Schafts 33. Entsprechendes gilt für einen Schaft 32 der Tibiakomponente 30.

Die erfindungsgemäße Gelenkprothese kann auch für andere Gelenke verwendet werden, beispielsweise am Ellenbogen oder an der Schulter.

Nachfolgend wird ein Weg zur Ausführung der Erfindung beschrieben.

Ausgangsmaterial ist eine β-Titanlegierung mit einem Molybdänanteil von 15 % (TiMo15). Diese Legierung kann handelsüblich in Form von kleinen Barren (Ingots) erworben werden.

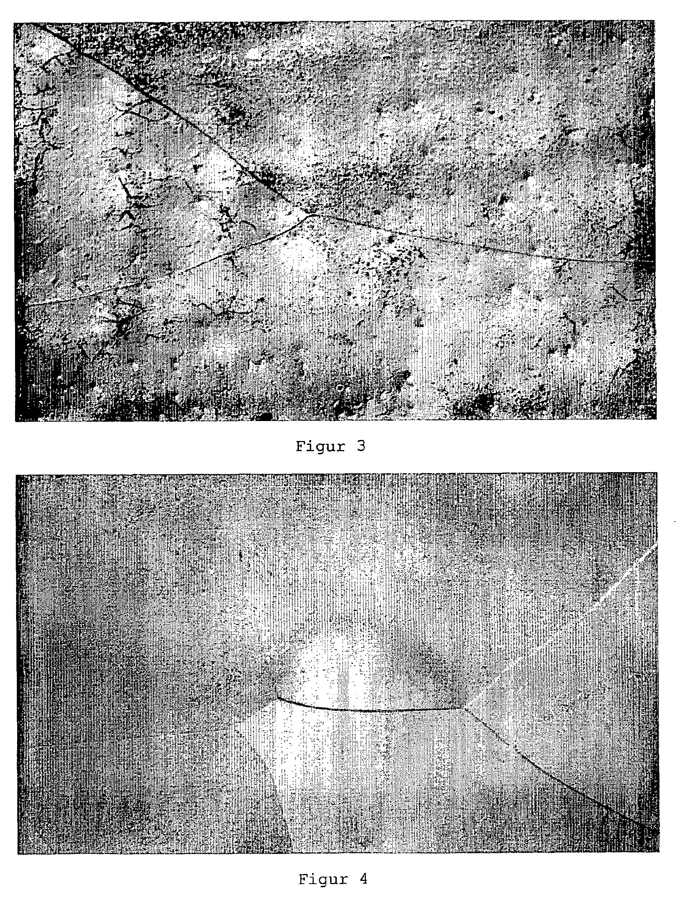

In einem ersten Schritt erfolgt ein Feingießen der Teile der Hüftprothese. Zum Schmelzen und Gießen des TiMo15 ist eine Gießanlage vorgesehen. Vorzugsweise handelt es sich um eine Kaltwandtiegel-Vakuuminduktions-Schmelz- und Gießanlage. Mit einer solchen Anlage können die hohen Temperaturen, die für ein sicheres Schmelzen von TiMo15 zum Feingießen erforderlich sind, erreicht werden. Der Schmelzpunkt von TiMo15 liegt bei 1770 °C zuzüglich eines Zuschlags von ca. 60 °C für ein sicheres Feingießen. Insgesamt muss also eine Temperatur von 1830 °C erreicht werden. Das Feingießen der Schmelze erfolgt anschließend mittels an sich bekannter Verfahren, beispielsweise mit Wachskernen und Keramikformen als verlorene Form. Derartige Feingusstechniken sind zum Feingießen von TiA16V4 bekannt. Es entsteht eine kubisch mittenzentrierte Kristallstruktur. Eine Abbildung des Gefüges ist in Fig. 3 dargestellt.

Die nach dem Feingießen von den Gießformen befreiten Gusskörper werden einer Wärmebehandlung unterzogen. Dazu ist ein heißisostatisches Pressen (HIP) bei einer Temperatur knapp unterhalb der β-Transustemperatur vorgesehen. Sie liegt im Bereich 710 °C bis 760 °C, vorzugsweise beträgt sie etwa 740 °C bei einem Argondruck von 1100 bis 1200 bar. Es ist zweckmäßig, dazu eine beim Giessen eventuell entstandene Randzone in Gestalt einer harten, spröden Schicht (sog. α-case) durch Beizen zu entfernen. Üblicherweise weist diese Schicht eine Dicke von ca. 0,03 mm auf.

Nach dem Hippen weisen die Gusskörper eine nur geringe Duktilität auf. Es wird vermutet, dass diese Versprödung auf sekundäre Ausscheidungen während des Hippen und der anschlie-βenden, in der Regel langsamen Abkühlung von der Hip-Temperatur zurückzuführen sind.

Um diese störenden Ausscheidungen in Lösung zu bringen, werden die Gusskörper in einem Kammerofen unter Argonschutzgasatmosphäre geglüht. Dazu wird ein Temperaturbereich von ca. 700 °C bis 860 °C gewählt, bei einer Dauer von mehreren, meist zwei Stunden. Es besteht hierbei ein gegenläufiger Zusammenhang zwischen der Temperatur und der Dauer, bei höherer Temperatur genügt eine kürzere Zeit und umgekehrt. Nach dem Lösungsglühen werden die Gusskörper mit kaltem Wasser abgeschreckt. Das sich dann ergebende Gefüge ist in Fig. 4 dargestellt.

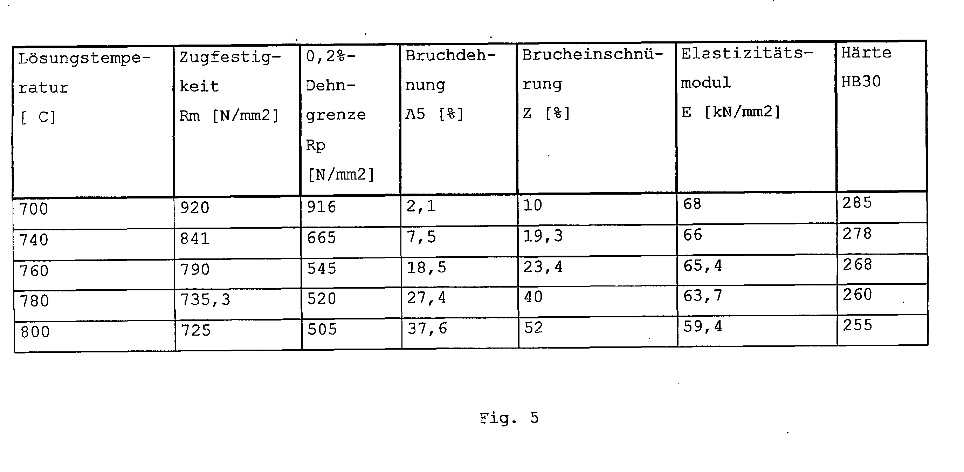

Die nach dem Lösungsglühen erreichten mechanischen Eigenschaften sind in der Tabelle in Fig. 5 wiedergegeben.

Man erkennt, dass der Elastizitätsmodul mit steigender Temperatur beim Lösungsglühen zurückgeht, und zwar auf Werte bis zu 60.000 N/mm². Die Zähigkeitswerte verbessern sich mit abnehmender Festigkeit und Härte. So erreicht man nach zweistündigem Lösungsglühen bei 800 °C einen Elastizitätsmodul von 60.000 N/mm² bei einer Bruchdehnung von ca. 40 % und einer Bruchfestigkeit Rm von ca. 730 N/mm².

## Patentansprüche

1. Gelenkprothese mit einem Schaft aus einer Titanlegierung,
**dadurch gekennzeichnet, dass**
zumindest der Schaft (10, 32, 33) feingegossen ist und eine mittenzentrierte kubische Kristallstruktur aufweist, dass die Titanlegierung eine Titan-Molybdän-Legierung mit einem Molybdängehalt von 15%, Rest Titan ist und dass der Elastizitätsmodul des Schafts 59,4 kN/mm² bis 68 kN/mm² beträgt.

2. Gelenkprothese nach Anspruch 1,
**dadurch gekennzeichnet, dass**
sie als Femurprothese (1) ausgeführt ist.

3. Gelenkprothese nach Anspruch 1,
**dadurch gekennzeichnet, dass**
sie als Knieprothese (3) ausgeführt ist.

4. Gelenkprothese nach einem der vorhergehenden Ansprüche,
**dadurch gekennzeichnet, dass**
die mittlere Korngröße der Kristallstruktur mindestens 0,3 mm, vorzugsweise 0,5 mm beträgt.

5. Gelenkprothese nach einem der vorhergehenden Ansprüche,
**dadurch gekennzeichnet, dass**
zumindest der Schaft (10, 32, 33) heißisostatisch gepresst und lösungsgeglüht ist.

6. Gelenkprothese nach Anspruch 5,
**dadurch gekennzeichnet, dass**
das heißisostatische Pressen bei einer Temperatur erfolgt ist, die maximal so hoch wie eine Beta-Transustemperatur der Titanlegierung und minimal 100 °C unter der Beta-Transustemperatur liegt.

7. Gelenkprothese nach einem der vorhergehenden Ansprüche,
**dadurch gekennzeichnet, dass**
die Titanlegierung vanadium- und aluminiumfrei ist.

8. Gelenkprothese nach einem der vorhergehenden Ansprüche;
**dadurch gekennzeichnet, dass**
die Titanlegierung bismutfrei ist.

## Claims

1. Joint prosthesis having a shaft made from a titanium alloy,
**characterized in that**
at least the shaft (10, 32, 33) is investment-cast and has a body-centered cubic crystal structure, **in that** the titanium alloy is a titanium-molybdenum alloy with a molybdenum content of 15%, remainder titanium, and **in that** the modulus of elasticity of the shaft is from 59.4 kN/mm² to 68 kN/mm².

2. Joint prosthesis according to Claim 1,
**characterized in that**
it is designed as a femoral prosthesis (1).

3. Joint prosthesis according to Claim 1,
**characterized in that**
it is designed as a knee prosthesis (3).

4. Joint prosthesis according to one of the preceding claims,
**characterized in that**
the mean grain size of the crystal structure is at least 0.3 mm, preferably 0.5 mm.

5. Joint prosthesis according to one of the preceding claims,
**characterized in that**
at least the shaft (10, 32, 33) is hot isostatically pressed and solution annealed.

6. Joint prosthesis according to Claim 5,
**characterized in that**
the hot isostatic pressing takes place at a temperature which is at most equal to a beta-transus temperature of the titanium alloy and is at most 100°C below the beta-transus temperature.

7. Joint prosthesis according to one of the preceding claims,
**characterized in that**
the titanium alloy is free of vanadium and aluminum.

8. Joint prosthesis according to one of the preceding claims,
**characterized in that**
the titanium alloy is free of bismuth.

## Revendications

1. Prothèse d'articulation dotée d'une tige en alliage de titane,
**caractérisée en ce que**
au moins la tige (10, 32, 33) est coulée finement et présente une structure cristalline cubique centrée,
**en ce que** l'alliage de titane est un alliage de titane et de molybdène dont la teneur en molybdène est de 15 %, le reste étant le titane et
**en ce que** le module d'élasticité de la tige est compris entre 59,4 kN/mm² et 68 kN/mm².

2. Prothèse d'articulation selon la revendication 1, **caractérisée en ce qu'**elle est configurée comme prothèse du fémur (1).

3. Prothèse d'articulation selon la revendication 1, **caractérisée en ce qu'**elle est configurée comme prothèse du genou (3).

4. Prothèse d'articulation selon l'une des revendications précédentes, **caractérisée en ce que** la taille moyenne des grains de la structure cristalline est d'au moins 0,3 mm et de préférence de 0,5 mm.

5. Prothèse d'articulation selon l'une des revendications précédentes, **caractérisée en ce qu'**au moins la tige (10, 32, 33) est comprimée en conditions isostatiques à chaud et subit un recuit de
solubilisation.

6. Prothèse d'articulation selon la revendication 5, **caractérisée en ce que** la compression isostatique à chaud est effectuée à une température située au maximum au niveau de la température de transition beta de l'alliage de titane et au minimum à 100°C en dessous de la température de transition beta.

7. Prothèse d'articulation selon l'une des revendications précédentes, **caractérisée en ce que** l'alliage de titane ne présente ni vanadium ni aluminium.

8. Prothèse d'articulation selon l'une des revendications précédentes, **caractérisée en ce que** l'alliage de titane est exempt de bismuth.
